# EUROPEAN PATENT APPLICATION

(11) **EP 3 384 947 A1**
(43) Date of publication of application: **10.10.2018**
(21) Application number: 17164825.6
(22) Date of filing: 04.04.2017
(51) Int. Cl.: A61M 16/00, A61M 15/00

(54) **FLUID DELIVERY DEVICE**

(71) Applicant: PARI GmbH Spezialisten für effektive Inhalation, 82319 Starnberg (DE)
(72) Inventor: Buchmann, Dr. Nicolas, 82008 Unterhaching (DE); Pirmann, Anton, 81243 Munich (DE); Schwenck, Dr. Nicolas, 81375 Munich (DE); Peter, Christian, 82239 Alling (DE)
(74) Representative: Hoffmann Eitle

(57) **Abstract**

The invention relates to a fluid delivery device (2, 2') for delivering a fluid into a human or animal body. The fluid delivery device (2, 2') comprises a fluid chamber (4, 4') for receiving a fluid, an oscillator (6, 6') for imparting oscillations to at least a portion of the fluid and a control for controlling the oscillator (6, 6'). The oscillator (6, 6') comprises a vibrator (8, 8') and a resonator (10, 10'). The resonator (10, 10') has a main body (12, 12'), defining an interior volume (14, 14'), and a neck (16, 16'). The neck (16, 16') is connected to the main body (12, 12') and in fluid communication with the interior volume (14, 14'). The vibrator (8, 8') is configured to impart vibrations to the resonator (10, 10'). The control is configured to control operation of the vibrator (8, 8') so as to impart vibrations to the resonator (10, 10') at a resonance frequency of the resonator (10, 10') or at a frequency which is within ± 20% of a resonance frequency of the resonator (10, 10'). The invention further relates to a method of operating the fluid delivery device (2, 2').

## Description

### Field of the Invention

The invention relates to a fluid delivery device for delivering a fluid into a human or animal body and to a method of operating the fluid delivery device. Further, the invention relates to an oscillator system for the fluid delivery device.

### Background Art

Diseases and conditions affecting either the paranasal sinuses or both the nasal cavity and the paranasal sinuses, in particular, acute and chronic forms of rhinosinusitis, are increasing in incidence and prevalence in many countries and regions of the world, including Europe and the United States. These conditions may be associated with significant symptoms and have a negative impact on quality of life and daily functioning.

However, while the mucosa of the nasal cavity is a feasible target for locally administered drugs formulated as nasal sprays, the sinuses and the osteomeatal complex are not easily accessed by liquid formulations. In the case of relatively coarse aerosols, such as conventional nasal sprays, the deposition on the sinus mucosa is negligible, and even finer aerosols, such as those generated by nebulisers, exhibit a very low degree of sinus deposition.

The primary reason for the lack of access of an inhaled aerosol to the sinuses is anatomical: in contrast to the nasal cavity, the sinuses are not actively ventilated. The latter are connected to the nasal passage via small orifices called ostia, whose diameter is typically in the region of only about 0.5 to 3.0 mm. When air is inhaled through the nose and passes through the nasal passage into the trachea, there is only very little convective flow into the ostia.
To address the need for devices and methods which are more effective in delivering an aerosol to the osteomeatal complex and paranasal sinuses, it was suggested in WO 2005/023335 that certain particle size and vorticity characteristics must be achieved in order that a majority of an aerosolised drug formulation reaches the deep nasal cavities and the sinuses. Furthermore, WO 2004/020029 discloses an aerosol generator comprising a nebuliser and a compressor which delivers a vibrating stream of air to the nebuliser. In use of this aerosol generator, the main aerosol flow supplied to a patient's nostril is superimposed by pressure fluctuations in order to improve the aerosol deposition efficiency in the paranasal sinuses. This document further describes that the aerosol emitted from the nebuliser should be introduced through one nostril via an appropriate nosepiece with closed soft palate, and that the contralateral nostril should be closed by an appropriate flow resistance device.

A substantial further improvement was achieved through the teaching of EP 1 820 493 A2 according to which the sinunasal deposition of a vibrating aerosol can be significantly increased if it is ensured that the pressure fluctuation maintains a certain amplitude, such as at least about 5 mbar pressure difference.

Superimposing the fluid flow by pressure oscillations or fluctuations not only helps to improve the fluid delivery efficiency in the paranasal sinuses but also allows for other regions of the human or animal body which are difficult to reach, for example, in the lungs, to be provided with a fluid, in particular, an aerosol containing fluid.

The fluid flow supplied to the patient's body is conventionally superimposed by pressure oscillations or fluctuations by means of mechanical oscillators, such as piston-actuated oscillators.

However, the characteristics of conventional oscillators, such as mechanical oscillators, in terms of bulkiness, noise generation and power consumption render their use in hand-held or portable devices unfeasible.

Hence, there is a need for a compact fluid delivery device which allows for the efficient delivery of a fluid into a human or animal body with reduced disturbance to the patient.

There is also a need for a method of operating such a fluid delivery device and for an oscillator system for such a fluid delivery device.

### Summary of the Invention

An object of the invention is to provide a compact fluid delivery device which allows for the efficient delivery of a fluid into a human or animal body with reduced disturbance to the patient. Further, the invention aims to provide a method of operating such a fluid delivery device and an oscillator system for such a fluid delivery device. These goals are achieved by a fluid delivery device with the technical features of claim 1, and a fluid delivery device with the technical features of claim 2, a method of operating the fluid delivery device with the technical features of claim 20 and an oscillator system for the fluid delivery device with the technical features of claim 21. Preferred embodiments of the invention follow from the dependent claims.

The invention provides, according to a first aspect, a fluid delivery device for delivering a fluid into a human or animal body. The fluid delivery device comprises a fluid chamber for receiving a fluid, an oscillator for imparting oscillations to at least a portion of the fluid and a control for controlling the oscillator. The oscillator comprises a vibrator and a resonator. The resonator has a main body, defining an interior volume, and a neck. The neck is connected to the main body and in fluid communication with the interior volume. The vibrator is configured to impart vibrations to the resonator. The control is configured to control operation of the vibrator so as to impart vibrations to the resonator at a resonance frequency of the resonator or at a frequency which is within ± 20% of a resonance frequency of the resonator.

The control may be configured to control operation of the vibrator so as to impart vibrations to the resonator at a resonance frequency of the resonator or at a frequency which is within ± 15% of a resonance frequency of the resonator. The control may be configured to control operation of the vibrator so as to impart vibrations to the resonator at a resonance frequency of the resonator or at a frequency which is within ± 10% of a resonance frequency of the resonator. The control may be configured to control operation of the vibrator so as to impart vibrations to the resonator at a resonance frequency of the resonator or at a frequency which is within ± 5% of a resonance frequency of the resonator.

The neck defines an inner space which, at one end thereof, is open to the outside of the resonator and, at the other end thereof, is open to the interior volume of the main body.

The end of the inner space of the neck which is open to the outside of the resonator may be in fluid communication with the fluid chamber.

The main body and the neck are arranged relative to each other along an arrangement direction. The neck is connected to the main body at a connection portion. A cross-section or cross-sectional area of the interior volume of the main body perpendicular to the arrangement direction at the connection portion is larger than an inner cross-section or cross-sectional area of the neck, i.e., a cross-section or cross-sectional area of the inner space defined by the neck, perpendicular to the arrangement direction at the connection portion. The inner cross-section or cross-sectional area of the resonator gradually or abruptly decreases at the connection portion.

The resonator may be a Helmholtz resonator.

The fluid is a substance that continually deforms or flows under an applied shear stress. The fluid may contain a liquid, a gas, a plasma or an aerosol.

The fluid may be a gas, such as air, air enriched with oxygen, or mixtures of any of helium, nitrogen, carbon, inert gases, water and oxygen.

An aerosol is a colloid of fine solid particles or liquid droplets, in air or another gas.

The terms "oscillation" and "vibration" define periodic changes of pressure that occur at a predetermined frequency. The oscillations may be regular, i.e., the time interval between pressure peaks may be approximately constant. The amplitude, i.e., the pressure amplitude, of the oscillations may be substantially constant. The vibrations may be regular, i.e., the time interval between pressure peaks may be approximately constant. The amplitude, i.e., the pressure amplitude, of the vibrations may be substantially constant.

The oscillations and/or vibrations may have more complex patterns, e.g., with longer on/off periods. The excitation of the oscillations and/or vibrations may be, for example, in the form of rectangular pulses.

The vibrator is configured to impart vibrations to the resonator. The vibrator is thus configured so as to impart vibrations to a fluid, e.g., a gas, such as for example air, received in the resonator, i.e., in the interior volume of the main body and/or the inner space of the neck.

The vibrations imparted to the resonator by the vibrator are amplified by the resonator. Oscillations are imparted to at least a portion of the fluid by the resonator of the oscillator. The oscillations are imparted through the end of the inner space of the neck which is open to the outside of the resonator.

The control is configured to control operation of the vibrator so as to impart vibrations to the resonator at a resonance frequency of the resonator or at a frequency which is within ± 20% of a resonance frequency of the resonator. A resonance frequency of the resonator is a frequency at which maximum amplification of the vibrations imparted to the resonator by the vibrator occurs. Therefore, by controlling operation of the vibrator as specified above, the vibrations imparted to the resonator are amplified in an efficient and reliable manner.

The control may be any type of control, e.g., a control unit, a control element, a control circuit or the like. For example, the control may comprise or be a computer, a processor, such as a microprocessor, a circuit or the like. The control may be connected to the vibrator, e.g., to a power supply element of the vibrator, for example, through one or more leads and/or electrical contacts.

The fluid delivery device according to the invention allows for oscillations to be imparted to at least a portion of the fluid in an efficient manner. Vibrations imparted to the resonator by the vibrator are reliably amplified by the resonator so that oscillations with high amplitudes can be imparted to at least a portion of the fluid without the need for using a mechanical oscillator, such as a piston-actuated oscillator. Hence, the fluid delivery device can be configured with a compact structure. Further, by using the resonator, having the main body and the neck, the noise generated when imparting the oscillations to the fluid can be minimised, thus minimising any possible disturbance to the patient.

The above configuration of the fluid delivery device further allows for the power consumption required for imparting the oscillations to at least a portion of the fluid to be considerably reduced. For this reason, and also due to the compact device configuration, the fluid delivery device of the invention can be particularly advantageously used as a hand-held or portable device.

The pressure amplitudes and the frequencies of the oscillations imparted to at least a portion of the fluid can be precisely set or adjusted within wide pressure and frequency ranges by suitably choosing the configuration of the resonator, in particular, the interior volume of the main body and the inner space of the neck. Moreover, these parameters can be efficiently varied by controlling the vibrations imparted to the resonator by the vibrator. The fluid delivery device of the invention thus offers a greater degree of freedom than conventional fluid delivery devices which rely on mechanical oscillators, such as piston-actuated oscillators.

The oscillations imparted to at least a portion of the fluid by the oscillator allow for fluid to be efficiently delivered also to regions of the human or animal body which are difficult to reach, such as the paranasal sinuses or some areas of the lungs.

The vibrator may be configured to impart vibrations to the main body. The vibrations may be imparted to a fluid, e.g., a gas, such as for example air, received in the interior volume of the main body. The control may be configured to control operation of the vibrator so as to impart vibrations to the main body at a resonance frequency of the resonator.

The vibrator may be configured to impart harmonic vibrations to the main body. The vibrator may be configured to impart disharmonic vibrations to the main body.

The pressure amplitude of the vibrations imparted to the resonator by the vibrator may be smaller than the pressure amplitude of the oscillations generated by the resonator.

The fluid delivery device may be configured so that, between the end of the inner space of the neck which is open to the outside of the resonator and the location where the oscillations are imparted to at least a portion of the fluid, no constriction is present in the device which has an inner cross-section or cross-sectional area which is smaller than the cross-section or cross-sectional area of the end of the inner space of the neck which is open to the outside of the resonator.

The vibrator may be configured to impart vibrations to the resonator, in particular, the main body thereof, along a direction which is substantially parallel to the arrangement direction of the main body and the neck.

The invention further provides, according to a second aspect, a fluid delivery device for delivering a fluid into a human or animal body. The fluid delivery device comprises a fluid chamber for receiving a fluid, an oscillator for imparting oscillations to at least a portion of the fluid and a control for controlling the oscillator. The oscillator comprises a vibrator and a resonator. The fluid chamber forms a main body of the resonator, defining an interior volume. The resonator has the main body and a neck. The neck is connected to the main body and in fluid communication with the interior volume. The vibrator is configured to impart vibrations to the resonator. The control is configured to control operation of the vibrator so as to impart vibrations to the resonator at a resonance frequency of the resonator or at a frequency which is within ± 20% of a resonance frequency of the resonator.

The control may be configured to control operation of the vibrator so as to impart vibrations to the resonator at a resonance frequency of the resonator or at a frequency which is within ± 15% of a resonance frequency of the resonator. The control may be configured to control operation of the vibrator so as to impart vibrations to the resonator at a resonance frequency of the resonator or at a frequency which is within ± 10% of a resonance frequency of the resonator. The control may be configured to control operation of the vibrator so as to impart vibrations to the resonator at a resonance frequency of the resonator or at a frequency which is within ± 5% of a resonance frequency of the resonator.

The fluid delivery device according to the second aspect provides the technical effects and advantages already described in detail above for the fluid delivery device according to the first aspect. Further, the fluid delivery device according to the second aspect allows for a particularly compact and simple device configuration to be achieved.

The features described above for the fluid delivery device according to the first aspect also apply to the fluid delivery device according to the second aspect. The features described in the following apply to the fluid delivery devices according to the first and second aspects.

The vibrator may be at least partly received within the fluid chamber. In this way, the compactness of the device structure can be further enhanced.

The oscillator of the fluid delivery devices of the invention may comprise a plurality of resonators. The resonators may be connected to each other in series. The resonators may be arranged so that a subsequent resonator amplifies the oscillations generated by a preceding resonator. For example, the neck of a preceding resonator may form the main body of a subsequent resonator. In some embodiments, the oscillator may comprise two resonators, three resonators, four resonators, or more than four resonators.

The fluid chamber may form the main body of one of the plurality of resonators, for example, a second, a third or a fourth resonator.

The oscillator may be configured to impart oscillations to a fluid flow of at least a portion of the fluid received in the fluid chamber.

A fluid flow of at least a portion of the fluid received in the fluid chamber can be induced by the respiration of a patient, i.e., by inhalation of the patient through the fluid delivery device. For this purpose, the fluid delivery device advantageously may further comprise an adaptation element for adaptation to the respiratory system of a human or animal body. The oscillator may be configured to impart oscillations to the fluid flow induced by the patient's respiration.

The fluid delivery device may comprise a fluid conveying element for inducing a fluid flow of at least a portion of the fluid received in the fluid chamber. The oscillator may be configured to impart oscillations to the fluid flow.

The fluid conveying element may be configured to convey at least a portion of the fluid outside the fluid delivery device. The fluid conveying element may be configured to convey at least a portion of the fluid to a desired location outside the device, such as the nasal cavity, the mucosa in the nose or the lungs of a patient.

The fluid conveying element may comprise or consist of a valve or a plurality of valves, such as inlet and/or outlet valves, provided in the fluid delivery device. The fluid conveying element may comprise or consist of a valve or a plurality of valves, e.g., an inlet valve or inlet valves, which is or are configured to induce a fluid flow. The valve or valves may be configured to induce the fluid flow by exploiting the vibrations imparted to the resonator by the vibrator.

The valve or valves may be arranged in or on the resonator, e.g., the main body and/or the neck. The valve or valves may be arranged in or on the vibrator. For example, if the vibrator comprises a vibratable membrane, the valve or valves may be arranged in or on the membrane.

The fluid conveying element may comprise or consist of a compressor, such as a gas compressor, a pump, such as a diaphragm pump or a piston pump, a turbine, an injector, a gas supply connector, a ventilator or the like.

The fluid delivery device may be an aerosol delivery device and the fluid may be or contain an aerosol. The aerosol may contain liquid droplets and/or solid particles.

The delivery devices of the invention allow for a particularly efficient aerosol treatment, enabling efficient delivery of the aerosol into the human or animal body, while minimising any disturbance to the patient.

For example, the principle of applying an oscillating aerosol for enhanced sinus deposition is described in WO 2004/020029.

The fluid delivery device may further comprise an aerosol generator for generating an aerosol.

The aerosol generator may be a vibrating membrane aerosol generator, such as a vibrating membrane nebuliser, e.g., an electronic vibrating membrane nebuliser, a jet nebuliser, an atomiser or the like. The aerosol generator may be an electronic nebuliser, e.g., a piezoelectrically driven nebuliser, i.e., a nebuliser driven by a piezoelectric element. In this case, the piezoelectric element may be arranged for vibrating or oscillating a vibratable member of the aerosol generator. The aerosol generator may be a nebuliser, an ultrasonic wave nebuliser, a nebuliser causing liquid to spray out of 2 nozzles for creating two colliding jets, a metered-dose inhaler (MDI), a dry powder inhaler(DPI)or a single-substance spray nozzle.

The aerosol may contain saline or salt.

The aerosol generated by the aerosol generator may be a pharmaceutical aerosol for the delivery of an active compound. An active compound is a natural, biotechnology-derived or synthetic compound or mixture of compounds useful for the diagnosis, prevention, management or treatment of a disease, condition or symptom of a human or an animal. Other terms which may be used as synonyms of active compounds include, for example, active ingredient, active pharmaceutical ingredient, drug substance, diagnostic material, drug, medicament and the like.

The active compound may be a drug substance or a medicament which is useful for the prevention, management, diagnosis or treatment of any disease, symptom or condition affecting the body cavities, the abdomen, the eyes, the intestine, the stomach, the nose, the sinuses, the osteomeatal complex, the mouth, the trachea, the lungs, the bronchia, the bronchioles, the alveoli and/or the respiratory tract.

Among the active compounds which may be useful for serving one of the purposes named previously and that may be used together with the present invention are, for example, substances selected from the group consisting of anti-inflammatory compounds, anti-infective agents, antiseptics, prostaglandins, endothelin receptor agonists, phosphodiesterase inhibitors, beta-2-sympathicomimetics, decongestants, vasoconstrictors, anticholinergics, immunoglobulins (e.g. Ig, IgG, IgA, IgM), immunomodulators, mucolytics, anti-allergic drugs, antihistaminics, mast-cell stabilising agents, tumor growth inhibitory agents, wound healing agents, local anaesthetics, antioxidants, oligonucleotides, peptides, proteins, vaccines, vitamins, plant extracts, cholinesterase inhibitors, vasoactive intestinal peptide, serotonin receptor antagonists, and heparins, glucocorticoids, anti-allergic drugs, antioxidants, vitamins, leucotriene antagonists, anti-infective agents, antibiotics, antifungals, antivirals, mucolytics, decongestants, antiseptics, cytostatics, immunomodulators, vaccines, wound healing agents, local anaesthetics, oligonucleotides, xanthin derived agents, peptides, proteins and plant extracts. Such compound may be used in the form of a suspension, a solution, a colloidal formulation (i.e., liposomal), etc.

Examples of potentially useful anti-inflammatory compounds are glucocorticoids and non-steroidal anti-inflammatory agents such as betamethasone, beclomethasone, budesonide, ciclesonide, dexamethasone, desoxymethasone, fluoconolone acetonide, fluocinonide, flunisolide, fluticasone, icomethasone, rofleponide, triamcinolone acetonide, fluocortin butyl, hydrocortisone, hydroxycortisone-17-butyrate, prednicarbate, 6-methylprednisolone aceponate, mometasone furoate, dehydroepiandrosterone-sulfate (DHEAS), elastane, prostaglandin, leukotriene, bradykinin antagonists, non-steroidal anti-inflammatory drugs (NSAIDs), such as ibuprofen including any pharmaceutically acceptable salts, esters, isomers, stereoisomers, diastereomers, epimers, solvates or other hydrates, prodrugs, derivatives, or any other chemical or physical forms of active compounds comprising the respective active moieties.

Examples of anti-infective agents, whose class or therapeutic category is herein understood as comprising compounds which are effective against bacterial, fungal, and viral infections, i.e. encompassing the classes of antimicrobials, antibiotics, antifungals, antiseptics, and antivirals, are
- penicillins, including benzylpenicillins (penicillin-G-sodium, clemizone penicillin, benzathine penicillin G), phenoxypenicillins (penicillin V, propicillin), aminobenzylpenicillins (ampicillin, amoxycillin, bacampicillin), acylaminopenicillins (azlocillin, mezlocillin, piperacillin, apalcillin), carboxypenicillins (carbenicillin, ticarcillin, temocillin), isoxazolyl penicillins (oxacillin, cloxacillin, dicloxacillin, flucloxacillin), and amiidine penicillins (mecillinam);
- cephalosporins, including cefazolins (cefazolin, cefazedone); cefuroximes (cefuroxim, cefamandole, cefotiam), cefoxitins (cefoxitin, cefotetan, latamoxef, flomoxef), cefotaximes (cefotaxime, ceftriaxone, ceftizoxime, cefmenoxime), ceftazidimes (ceftazidime, cefpirome, cefepime), cefalexins (cefalexin, cefaclor, cefadroxil, cefradine, loracarbef, cefprozil), and cefiximes (cefixime, cefpodoxim proxetile, cefuroxime axetil, cefetamet pivoxil, cefotiam hexetil), loracarbef, cefepim, clavulanic acid / amoxicillin, Ceftobiprole;
- synergists, including beta-lactamase inhibitors, such as clavulanic acid, sulbactam, and tazobactam;
- carbapenems, including imipenem, cilastin, meropenem, doripenem, tebipenem, ertapenem, ritipenam, and biapenem;
- monobactams, including aztreonam;
- aminoglycosides, such as apramycin, gentamicin, amikacin, isepamicin, arbekacin, tobramycin, netilmicin, spectinomycin, streptomycin, capreomycin, neomycin, paromoycin, and kanamycin;
- macrolides, including erythromycin, clarythromycin, roxithromycin, azithromycin, dithromycin, josamycin, spiramycin and telithromycin;
- gyrase inhibitors or fluroquinolones, including ciprofloxacin, gatifloxacin, norfloxacin, ofloxacin, levofloxacin, perfloxacin, lomefloxacin, fleroxacin, garenoxacin, clinafloxacin, sitafloxacin, prulifloxacin, olamufloxacin, caderofloxacin, gemifloxacin, balofloxacin, trovafloxacin, and moxifloxacin;
- tetracyclins, including tetracyclin, oxytetracyclin, rolitetracyclin, minocyclin, doxycycline, tigecycline and aminocycline;
- glycopeptides, inlcuding vancomycin, teicoplanin, ristocetin, avoparcin, oritavancin, ramoplanin, and peptide 4;
- polypeptides, including plectasin, dalbavancin, daptomycin, oritavancin, ramoplanin, dalbavancin, telavancin, bacitracin, tyrothricin, neomycin, kanamycin, mupirocin, paromomycin, polymyxin B and colistin;
- sulfonamides, including sulfadiazine, sulfamethoxazole, sulfalene, co-trimoxazole, co-trimetrol, co-trimoxazine, and co-tetraxazine;
- azoles, including clotrimazole, oxiconazole, miconazole, ketoconazole, itraconazole, fluconazole, metronidazole, tinidazole, bifonazol, ravuconazol, posaconazol, voriconazole, and ornidazole and other antifungals including flucytosin, griseofulvin, tolnaftal, naftifin, terbinafin, amorolfin, ciclopiroxolamin, echinocandins, such as micafungin, caspofungin, anidulafungin;
- nitrofurans, including nitrofurantoin and nitrofuranzone;
- polyenes, including amphotericin B, natamycin, nystatin, flucytosine;
- other antibiotics, including tithromycin, lincomycin, clindamycin, oxazolindiones (linzezolids), ranbezolid, streptogramine A+B, pristinamycin A+B, Virginiamycin A+B, dalfopristin /quinupristin (Synercid), chloramphenicol, ethambutol, pyrazinamid, terizidon, dapson, prothionamid, fosfomycin, fucidinic acid, rifampicin, isoniazid, cycloserine, terizidone, ansamycin, lysostaphin, iclaprim, mirocin B17, clerocidin, filgrastim, formycin, and pentamidine;
- antivirals, including aciclovir, ganciclovir, birivudin, valaciclovir, zidovudine, didanosin, thiacytidin, stavudin, lamivudin, zalcitabin, ribavirin, nevirapirin, delaviridin, trifluridin, ritonavir, saquinavir, indinavir, foscarnet, amantadin, podophyllotoxin, vidarabine, tromantadine, and proteinase inhibitors, siRNA based drugs;
- antiseptics, including acridine derivatives, iodine-povidone, benzoates, rivanol, chlorhexidine, quarternary ammonium compounds, cetrimides, biphenylol, clorofene, and octenidine;
- plant extracts or ingredients, such as plant extracts from chamomile, hamamelis, echinacea, calendula, thymian, papain, pelargonium, pine trees, essential oils, myrtol, pinen, limonen, cineole, thymol, mentol, camphor, tannin, alpha-hederin, bisabolol, lycopodin, vitapherole;
- wound healing compounds including dexpantenol, allantoin, vitamins, hyaluronic acid, alpha-antitrypsin, anorganic and organic zinc salts/compounds, salts of bismuth and selen;
- interferones (alpha, beta, gamma), tumor necrosis factors, cytokines, interleukines;
- immunmodulators including methotrexat, azathioprine, cyclosporine, tacrolimus, sirolimus, rapamycin, mofetil; mofetil-mycophenolate.
- cytostatics and metastasis inhibitors;
- alkylants, such as nimustine, melphanlane, carmustine, lomustine, cyclophosphosphamide, ifosfamide, trofosfamide, chlorambucil, busulfane, treosulfane, prednimustine, thiotepa;
- antimetabolites, e.g. cytarabine, fluorouracil, methotrexate, mercaptopurine, tioguanine;
- alkaloids, such as vinblastine, vincristine, vindesine;
- antibiotics, such as alcarubicine, bleomycine, dactinomycine, daunorubicine, doxorubicine, epirubicine, idarubicine, mitomycine, plicamycine;
- complexes of transition group elements (e.g. Ti, Zr, V, Nb, Ta, Mo, W, Pt) such as carboplatinum, cis-platinum and metallocene compounds such as titanocendichloride;
- amsacrine, dacarbazine, estramustine, etoposide, beraprost, hydroxycarbamide, mitoxanthrone, procarbazine, temiposide;
- paclitaxel, gefitinib, vandetanib, erlotinib, poly-ADP-ribose-polymerase (PRAP) enzyme inhibitors, banoxantrone, gemcitabine, pemetrexed, bevacizumab, ranibizumab. Examples of potentially useful mucolytics are DNase, P2Y2-agonists (denufosol), drugs affecting chloride and sodium permeation, such as N-(3,5-Diamino-6-chloropyrazine-2-carbony)-N'-{4-[4-(2,3-dihydroxypropoxy)-phenyl]butyl}guanidine methanesulfonate (PARION 552-02), heparinoids, guaifenesin, acetylcysteine, carbocysteine, ambroxol, bromhexine, tyloxapol, lecithins, myrtol, surfactant, and recombinant surfactant proteins.

Examples of potentially useful vasoconstrictors and decongestants which may be useful to reduce the swelling of the mucosa are phenylephrine, naphazoline, tramazoline, tetryzoline, oxymetazoline, fenoxazoline, xylometazoline, epinephrine, isoprenaline, hexoprenaline, and ephedrine. Examples of potentially useful local anaesthetic agents include benzocaine, tetracaine, procaine, lidocaine and bupivacaine.

Examples of potentially useful antiallergic agents include the afore-mentioned glucocorticoids, cromolyn sodium, nedocromil, cetrizin, loratidin, montelukast, roflumilast, ziluton, omalizumab, heparinoids and other antihistamins, including azelastine, cetirizin, desloratadin, ebastin, fexofenadin, levocetirizin, loratadin.

Examples of potentially useful anticholinergic agents include ipratropium bromide, tiotropium bromide, oxitropium bromide, glycopyrrolate.

Examples of potentially useful beta-2-sympathicomimetic agents include salbutamol, fenoterol, formoterol, indacaterol, isoproterenol, metaproterenol, salmeterol, terbutaline, clenbuterol, isoetarine, pirbuterol, procaterol, ritodrine.

Examples of xanthine derived agents include theophylline, theobromine, caffeine.

Antisense oligonucleotides are short synthetic strands of DNA (or analogs) that are complimentary or antisense to a target sequence (DNA, RNA) designed to halt a biological event, such as transcription, translation or splicing. The resulting inhibition of gene expression makes oligonucleotides dependent on their composition useful for the treatment of many diseases and various compounds are currently clinically evaluated, such as ALN-RSV01 to treat the respiratory syncytical virus by, AVE-7279 to treat asthma and allergies, TPI-ASM8 to treat allergic asthma, 1018-ISS to treat cancer. Examples of potentially useful peptides and proteins include antibodies against toxins produced by microorganisms, antimicrobial peptides such as cecropins, defensins, thionins, and cathelicidins.

The vibrator may comprise a vibratable membrane. The control may be configured to control operation of the vibratable membrane so as to impart vibrations to the resonator, in particular, the main body thereof, at a resonance frequency of the resonator or at a frequency which is within ± 20% of a resonance frequency of the resonator.

The main body of the resonator may comprise a vibratable membrane.

The vibratable membrane may be a continuous membrane, i.e., a membrane which does not have openings or holes that fully penetrate the membrane formed therein.

The use of a vibratable membrane for generating vibrations allows for a particularly compact, quiet and flexible configuration of the fluid delivery device to be achieved.

It has been difficult or even impossible to achieve oscillations with high pressure amplitudes using vibratable membranes. However, this problem has been overcome by the present invention, using a resonator for amplifying vibrations imparted thereto by the vibrator. Hence, at least a portion of the fluid can be efficiently oscillated by the oscillator comprising the vibrator and the resonator while, at the same time, ensuring a compact, quiet and flexible device configuration.

The vibratable membrane of the vibrator or the main body of the resonator may be driven by an electromagnet, such as a coil, a piezoelectric element, a crank mechanism or the like. For example, the vibrator may be substantially in the form of a loudspeaker.

The vibrator may be configured to impart vibrations to the resonator, in particular, the main body, by exploiting pressure oscillations which are present in other parts of the fluid delivery device, for example, for the case of a vibrating membrane aerosol generator, comprising a perforated vibratable membrane, or are present in a related system. If a compressor is used for fluid transport and/or aerosol generation, pressure pulsations of the compressor may be used for imparting vibrations to the resonator, in particular, the main body, e.g., during exhalation phases of a patient.

The control may be configured to control operation of the vibrator so as to impart vibrations to the resonator, in particular, the main body thereof, at a constant frequency. The constant frequency may be a resonance frequency of the resonator or a frequency which is within ± 20% of a resonance frequency of the resonator. In this case, a particularly efficient amplification of the vibrations imparted to the resonator can be ensured.

The constant frequency may be a resonance frequency of the resonator or a frequency which is within ± 15% of a resonance frequency of the resonator. The constant frequency may be a resonance frequency of the resonator or a frequency which is within ± 10% of a resonance frequency of the resonator. The constant frequency may be a resonance frequency of the resonator or a frequency which is within ± 5% of a resonance frequency of the resonator.

The control may be configured to control operation of the vibrator so as to impart vibrations to the resonator, in particular, the main body thereof, at a plurality of different frequencies. At least one of the plurality of frequencies may be a resonance frequency of the resonator or a frequency which is within ± 20% of a resonance frequency of the resonator.

At least one of the plurality of frequencies may be a resonance frequency of the resonator or a frequency which is within ± 15% of a resonance frequency of the resonator. At least one of the plurality of frequencies may be a resonance frequency of the resonator or a frequency which is within ± 10% of a resonance frequency of the resonator. At least one of the plurality of frequencies may be a resonance frequency of the resonator or a frequency which is within ± 5% of a resonance frequency of the resonator.

The control may be configured to control operation of the vibrator so as to impart vibrations to the resonator, in particular, the main body, at a plurality of different frequencies which are varied, e.g., decreased and/or increased, over time. In particular, the control may be configured to control operation of the vibrator so as to scan through a frequency band, in particular, so as to continuously scan through a frequency band, wherein the frequency band comprises one or more resonance frequencies of the resonator and/or one or more frequencies which are within ± 20% of a resonance frequency of the resonator.

Imparting vibrations to the resonator, in particular, the main body, at a plurality of different frequencies, in particular, in the form of one or more frequency bands, offers the advantage that regions of the human or animal body which can be most efficiently reached with oscillations having different frequencies, e.g., due to different diameters thereof, can be reliably supplied with fluid. Since the diameters of such regions, e.g., the ostia, will also vary from patient to patient, particularly efficient and reliable fluid delivery can be ensured independent of the specific anatomy of the patient.

The resonator may have a single resonance frequency or a plurality of resonance frequencies. The number and the value or values of the resonance frequencies can be adjusted by suitably configuring the shapes and/or relative dimensions of the main body and the neck of the resonator. The resonator may have two resonance frequencies, three resonance frequencies, four resonance frequencies, or more than four resonance frequencies.

If the resonator has a plurality of resonance frequencies, the flexibility of the fluid delivery device is further enhanced. In particular, in this way, maximum pressure amplitudes can be generated at different frequencies. Further, different possible amplifications are provided in a particularly efficient manner, for example, a weaker amplification for children or animals and a stronger amplification for adults.

The control may be configured to control operation of the vibrator so as to impart vibrations to the resonator, in particular, the main body, at different resonance frequencies of the resonator and/or at different frequencies which are within ± 20% of a resonance frequency of the resonator. The control may be configured to control operation of the vibrator so as to impart vibrations to the resonator, in particular, the main body, at a plurality of resonance frequencies and/or at a plurality of frequencies which are within ± 20% of a resonance frequency of the resonator, e.g., two such frequencies, three such frequencies, four such frequencies, or more than four such frequencies, e.g., resonance frequencies, of the resonator. The control may be configured to scan through a frequency band which comprises a plurality of resonance frequencies and/or a plurality of frequencies which are within ± 20% of a resonance frequency of the resonator, e.g., two such frequencies, three such frequencies, four such frequencies, or more than four such frequencies, e.g., resonance frequencies, of the resonator.

An inner cross-section or cross-sectional area of the neck, perpendicular to a fluid path direction in the neck, may be substantially constant along the fluid path direction in the neck. The fluid path direction in the neck is the direction along which a fluid can pass from the one end of the inner space of the neck which is open to the interior volume of the main body to the other end of the inner space which is open to the outside of the resonator.

The main body and the neck are arranged relative to each other along an arrangement direction. An inner cross-section or cross-sectional area, perpendicular to the arrangement direction, of the neck may be substantially constant along the arrangement direction.

The inner space of the neck may have a cylindrical shape, a prism shape, an ellipsoidal shape or the like. Alternatively, the inner space of the neck may have any other type of shape, for example, an irregular shape.

The neck may have a straight shape or a bent or curved shape. The interior volume of the main body may have a cylindrical shape or a spherical shape. Alternatively, the interior volume of the main body may have any other type of shape, for example, an irregular shape.

A length of the neck in or along the fluid path direction in the neck may be in the range of 15 mm to 150 mm, preferably 25 mm to 75 mm.

A length of the neck in or along the arrangement direction may be in the range of 15 mm to 150 mm, preferably 25 mm to 75 mm.

An inner diameter, e.g., an equivalent inner diameter, of the neck, perpendicular to the fluid path direction in the neck, may be in the range of 1.0 mm to 30.0 mm, preferably 5.0 mm to 20.0 mm.

An inner diameter, e.g., an equivalent inner diameter, of the neck, perpendicular to the arrangement direction, may be in the range of 1.0 mm to 30.0 mm, preferably 5.0 mm to 20.0 mm.

If the cross-sectional area of the end of the inner space of the neck which is open to the outside of the resonator is denoted by A, the length of the neck in the fluid path direction or the arrangement direction is denoted by L, and the size of the interior volume of the main body is denoted by V, the parameter A/(VxL) may be in the range of 0 to 5000 m⁻², preferably 0.1 to 400 m⁻² and more preferably 1 to 400 m⁻². Particularly preferably, the parameter A/(VxL) may be in the range of 0.1 to 100 m⁻², even more preferably 1 to 100 m⁻².

The size of the interior volume of the main body may be in the range of 10 ml to 800 ml, preferably 50 ml to 400 ml.

The resonator may have at least one resonance frequency in the range of 10 Hz to 200 Hz, preferably 30 Hz to 150 Hz and more preferably 50 Hz to 100 Hz. Particularly preferably, the resonator may have at least one resonance frequency in the range of 20 Hz to 100 Hz.

The resonator may have a plurality of resonance frequencies, e.g., two resonance frequencies, three resonance frequencies, four resonance frequencies, or more than four resonance frequencies, in the range of 10 Hz to 200 Hz, preferably 30 Hz to 150 Hz and more preferably 50 Hz to 100 Hz. Particularly preferably, the resonator may have a plurality of resonance frequencies, e.g., two resonance frequencies, three resonance frequencies, four resonance frequencies, or more than four resonance frequencies, in the range of 20 Hz to 100 Hz.

The fluid delivery device may further comprise an adaptation element for adaptation to the respiratory system of a human or animal body. In this way, fluid can be delivered into the human or animal body in a particularly simple and efficient manner, in particular, for the case of a hand-held or portable device.

The oscillator may be removably or detachably attached to a remainder of the fluid delivery device. In this case, a single oscillator can be used for a plurality of fluid delivery devices, thus offering a particularly cost-efficient arrangement.

Alternatively, the oscillator may be integrally formed with a remainder of the fluid delivery device. For example, interior volumes or inner spaces of the fluid delivery device may be adapted or modified, e.g., partitioned, so as to form the resonator comprising the main body and the neck. In some embodiments, a portion of a fluid chamber of a fluid delivery device may be partitioned so as to form the interior volume of the main body of the resonator. In some embodiments, the vibrator may be at least partly received within the fluid chamber.

The vibrator may be removably or detachably attached to a remainder of the fluid delivery device. Alternatively, the vibrator may be integrally formed with a remainder of the fluid delivery device.

The main body of the resonator may have an inlet valve. The main body of the resonator may have one or more valves and/or openings for adjusting amplification of the vibrations imparted to the resonator, in particular, the main body, by the vibrator.

The invention further provides a method of operating the fluid delivery device of the invention. The method comprises controlling operation of the vibrator so as to impart vibrations to the resonator, in particular, the main body thereof, at a resonance frequency of the resonator or at a frequency which is within ± 20% of a resonance frequency of the resonator.

The method may comprise controlling operation of the vibrator so as to impart vibrations to the resonator at a resonance frequency of the resonator or at a frequency which is within ± 15% of a resonance frequency of the resonator. The method may comprise controlling operation of the vibrator so as to impart vibrations to the resonator at a resonance frequency of the resonator or at a frequency which is within ± 10% of a resonance frequency of the resonator. The method may comprise controlling operation of the vibrator so as to impart vibrations to the resonator at a resonance frequency of the resonator or at a frequency which is within ± 5% of a resonance frequency of the resonator.

The method of the invention is a method of operating the fluid delivery device of the invention. The method thus provides the technical effects and advantages already described in detail above for the fluid delivery device.

The features described above for the fluid delivery device of the invention also apply to the method of the invention.

The method may further comprise inducing a fluid flow of at least a portion of the fluid received in the fluid chamber. The method may comprise imparting oscillations to the fluid flow.

The method may further comprise generating an aerosol.

The method may comprise controlling operation of the vibrator so as to impart vibrations to the resonator, in particular, the main body, at a constant frequency. The constant frequency may be a resonance frequency of the resonator or a frequency which is within ± 20% of a resonance frequency of the resonator.

The method may comprise controlling operation of the vibrator so as to impart vibrations to the resonator, in particular, the main body, at a plurality of different frequencies, wherein at least one of the plurality of frequencies is a resonance frequency of the resonator or a frequency which is within ± 20% of a resonance frequency of the resonator. In particular, the method may comprise scanning a frequency band comprising one or more resonance frequencies of the resonator and/or one or more frequencies which are within ± 20% of a resonance frequency of the resonator.

The invention further provides an oscillator system for the fluid delivery device according to the invention. The oscillator system comprises an oscillator for imparting oscillations to at least a portion of the fluid, and a control for controlling the oscillator. The oscillator comprises a vibrator and a resonator. The resonator has a main body, defining an interior volume, and a neck. The neck is connected to the main body and in fluid communication with the interior volume. The vibrator is configured to impart vibrations to the resonator. The control is configured to control operation of the vibrator so as to impart vibrations to the resonator at a resonance frequency of the resonator or at a frequency which is within ± 20% of a resonance frequency of the resonator.

The control may be configured to control operation of the vibrator so as to impart vibrations to the resonator at a resonance frequency of the resonator or at a frequency which is within ± 15% of a resonance frequency of the resonator. The control may be configured to control operation of the vibrator so as to impart vibrations to the resonator at a resonance frequency of the resonator or at a frequency which is within ± 10% of a resonance frequency of the resonator. The control may be configured to control operation of the vibrator so as to impart vibrations to the resonator at a resonance frequency of the resonator or at a frequency which is within ± 5% of a resonance frequency of the resonator.

The oscillator system of the invention is an oscillator system for the fluid delivery device of the invention. The oscillator system thus provides the technical effects and advantages already described in detail above for the fluid delivery device.

The features described above for the fluid delivery device of the invention also apply to the oscillator system of the invention.

### Brief Description of the Drawings

Hereinafter, non-limiting examples of the present invention are explained with reference to the drawings, in which:
- Figure 1: shows a fluid delivery device according to a first embodiment of the present invention, wherein Figure 1(a) is a front view of the fluid delivery device, and Figure 1(b) is a partial cross-sectional view of the fluid delivery device taken along the line A-A in Figure 1(a);
- Figure 2: shows a fluid delivery device according to a second embodiment of the present invention, wherein Figure 2(a) is a front view of the fluid delivery device, and Figure 2(b) is a partial cross-sectional view of the fluid delivery device taken along the line A-A in Figure 2(a);
- Figure 3: is a diagram showing measurement results for the vibration pressure amplitude as a function of the vibration frequency for the fluid delivery device of the first embodiment; and
- Figure 4: is a diagram showing measurement results for the vibration pressure amplitude as a function of the electric power applied to the vibrator for the fluid delivery device of the first embodiment.

### Detailed Description of Currently Preferred Embodiments

Currently preferred embodiments of the present invention will now be described with reference to the accompanying drawings. The preferred embodiments relate to fluid delivery devices and to methods of operating these devices.

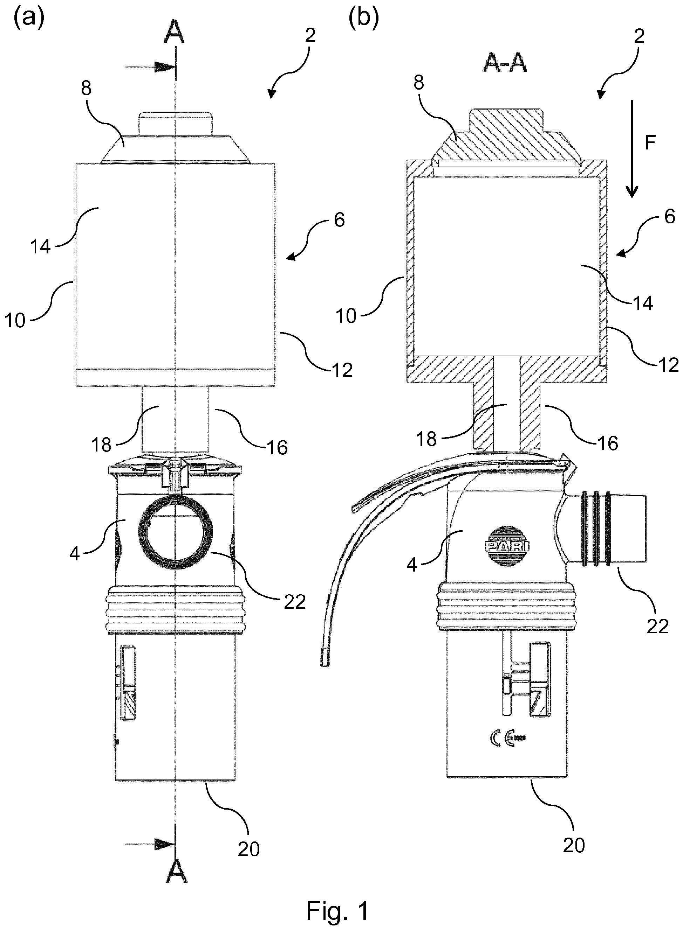

In the following, a first embodiment of the fluid delivery device of the present invention and of the operating method of the present invention will be described with reference to Figure 1.

Figure 1 shows a fluid delivery device 2 for delivering a fluid into a human or animal body according to a first embodiment of the present invention.

The fluid delivery device 2 comprises a fluid chamber 4 for receiving a fluid, an oscillator 6 for imparting oscillations to at least a portion of the fluid, and a control (not shown) for controlling the oscillator 6. The control may be, for example, a computer, a processor, such as a microprocessor, a circuit or the like.

The oscillator 6 comprises a vibrator 8 and a resonator 10. The resonator 10 has a main body 12, defining an interior volume 14, and a neck 16, as is shown in Figures 1(a) and (b). The neck 16 is connected to the main body 12 and in fluid communication with the interior volume 14 (see Figure 1(b)). The neck 16 defines an inner space 18 which, at one end thereof, is open to the interior volume 14 and, at the other end thereof, is open to the outside of the resonator 10. The interior volume 14 of the main body 12 and the inner space 18 of the neck 16 each have a cylindrical shape.

The interior volume 14 is in fluid communication with the fluid chamber 4 through the inner space 18 of the neck 16.

The fluid delivery device 2 is an aerosol delivery device for delivering an aerosol as the fluid into a human or animal body. Specifically, the fluid delivery device 2 is a jet nebuliser or atomiser in which an aerosol is generated by causing a compressed gas, such as air or oxygen, to exert aerodynamic forces on a liquid drug or medicament to be aerosolised. The compressed gas may be provided by a compressor (not shown). The fluid delivery device 2 has a connection 20 through which the compressed gas is supplied.

The oscillator 6, comprising the vibrator 8 and the resonator 10, is removably or detachably attached to a remainder of the fluid delivery device 2. For example, the oscillator 6 may be removably or detachably attached to a PARI LC Sprint nebuliser.

The oscillator 6 and the control together form an embodiment of the oscillator system of the present invention.

An aerosol or fluid comprising the gas and the aerosolised drug or medicament is received in the fluid chamber 4. From the fluid chamber 4, the aerosol or fluid is transported outside the device 2 into a human or animal body through a mouthpiece or nosepiece 22. The mouthpiece or nosepiece 22 is configured for adaptation to the respiratory system of the human or animal body.

A fluid flow for transporting at least a portion of the fluid received in the fluid chamber 4 outside the device 2 is induced by the member or component providing the compressed gas for aerosol generation, such as a compressor. Hence, in the fluid delivery device 2 of the first embodiment, this member or component serves as a fluid conveying element. The oscillator 6 is configured to impart oscillations to the fluid flow.

In the interior volume 14 and the inner space 18, a fluid, e.g., a gas, such as air, is received. The fluid can pass through the inner space 18 in a fluid path direction F (see Figure 1(b)) which is the direction from the one end of the inner space 18, which is open to the interior volume 14, to the other end of the inner space 18, which is open to the outside of the resonator 10. The fluid path direction F coincides with an arrangement direction along which the main body 12 and the neck 16 are arranged relative to each other.

An inner cross-section of the neck 16, i.e., a cross-section of the inner space 18, perpendicular to the fluid path direction F is significantly smaller than a cross-section of the interior volume 14 perpendicular to the fluid path direction F (see Figure 1(b)).

The vibrator 8 is configured to impart vibrations to the resonator 10, in particular, the main body 12. The vibrator 8 comprises a vibratable membrane (not shown). The vibratable membrane is a continuous membrane, i.e., a membrane which does not have openings or holes that fully penetrate the membrane formed therein. The vibratable membrane is arranged so that it substantially lies in a plane which is perpendicular to the fluid path direction F. Thus, the direction along which the vibrations are imparted to the resonator 10 by the vibrator 8 is parallel to the fluid path direction F.

In other embodiments, the vibratable membrane may be arranged so that it substantially lies in a plane which is parallel to the fluid path direction F.

For example, the vibrator 8 may be substantially in the form of a loudspeaker.

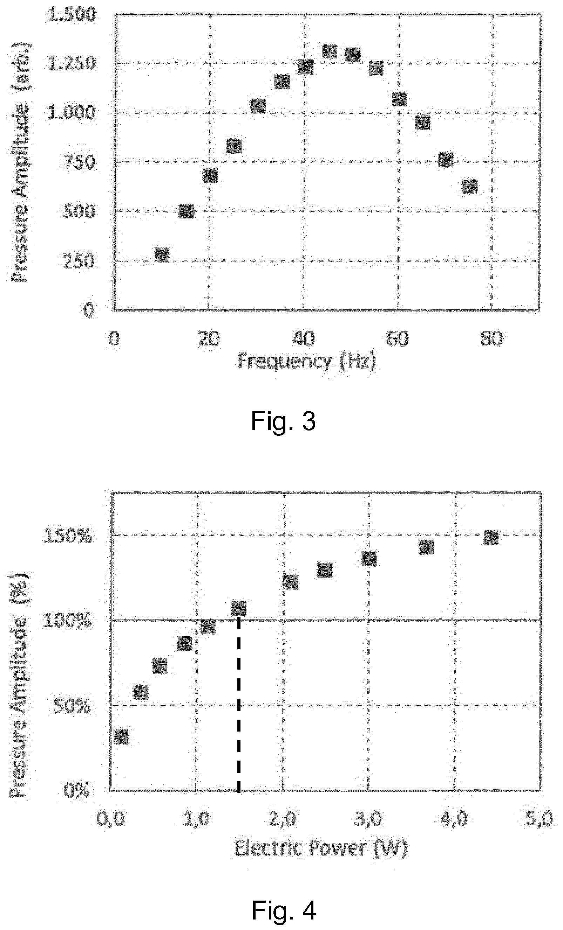

The vibrator 8 imparts vibrations to the main body 12, thus vibrating the fluid, such as air, received in the interior volume 14. The resonator 10, having the main body 12 and the neck 16, is a Helmholtz resonator having a predetermined resonance frequency. The resonance frequency of the resonator 10 is approximately 45 Hz (see Figure 3).

The control is configured to control operation of the vibrator 8 so as to impart vibrations to the Helmholtz resonator 10 at the resonance frequency thereof or at a frequency which is within ± 20% of the resonance frequency thereof. Thus, the vibrations imparted by the vibrator 8 are amplified by the resonator 10 through acoustic resonance. The amplified vibrations are used to oscillate the fluid flow, i.e., to impart oscillations to the fluid flow. The oscillations are imparted to the fluid flow through the end of the inner space 18 which is open to the outside of the resonator 10. This end of the inner space 18 is in fluid communication with the fluid chamber 4.

The control may be configured to control operation of the vibrator 8 so as to impart vibrations to the main body 12 at a constant frequency, namely the resonance frequency of the resonator 10 or a frequency which is within ± 20% of the resonance frequency of the resonator 10, or at a plurality of different frequencies, wherein at least one of the frequencies is the resonance frequency of the resonator 10 or a frequency which is within ± 20% of the resonance frequency of the resonator 10. In particular, the control may be configured to scan a frequency band comprising the resonance frequency of the resonator 10 or a frequency which is within ± 20% of the resonance frequency of the resonator 10.

The length of the neck 16 in the fluid path direction F is approximately 50 mm. The inner diameter of the neck 16, perpendicular to the fluid path direction F, is approximately 8 mm. The size of the interior volume 14 of the main body 12 is approximately 100 ml.

In operation of the fluid delivery device 2, the compressed gas is supplied to the device 2 through the connection 20 and produces droplets of a saline, liquid drug or medicament received in the device 2. The aerosol or fluid containing the gas and the droplets of saline, drug or medicament is received in the fluid chamber 4 and transported outside the device through the mouthpiece or nosepiece 22 by the fluid flow induced by the member or component providing the compressed gas, such as a compressor.

Operation of the vibrator 8 is controlled so as to impart vibrations to the main body 12 at the resonance frequency of the resonator 10 or at a frequency which is within ± 20% of the resonance frequency of the resonator 10. The vibrations imparted to the main body 12 are amplified by the resonator 10, so that oscillations with a significantly increased pressure amplitude are imparted to the flow of the fluid containing the aerosolised drug or medicament.

In this way, it can be ensured that the aerosolised drug or medicament is also supplied to regions of the human or animal body which otherwise are difficult to reach, such as the paranasal sinuses or some areas of the lungs.

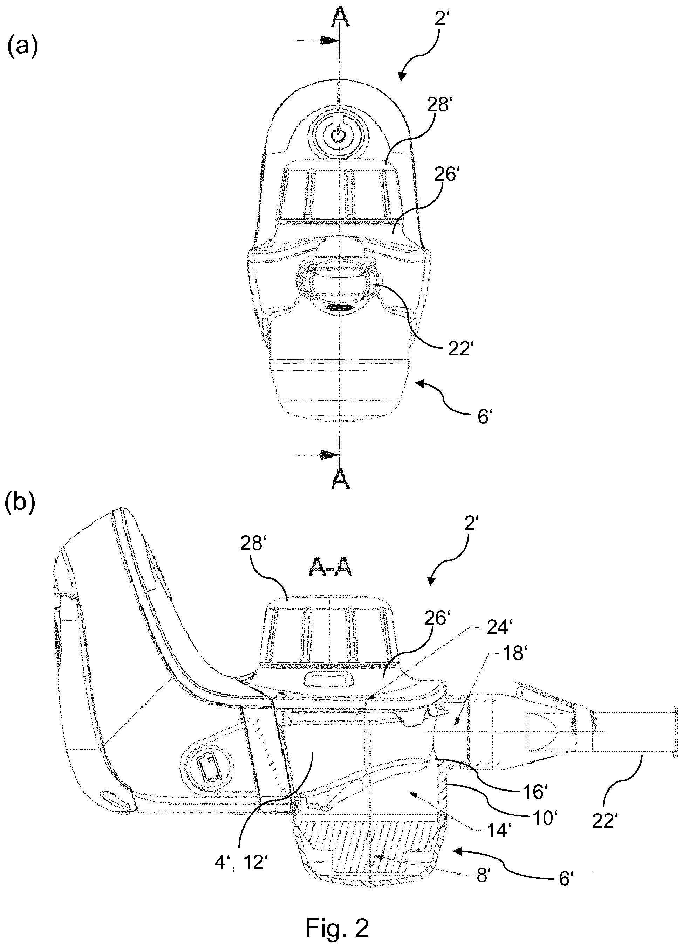

In the following, a second embodiment of the fluid delivery device of the present invention and of the operating method of the present invention will be described with reference to Figure 2.

The second embodiment of the invention substantially differs from the first embodiment of the invention in that the oscillator is integrally formed with a remainder of the fluid delivery device and the aerosol is generated in a different manner, as will be detailed below.

Figure 2 shows a fluid delivery device 2' for delivering a fluid into a human or animal body according to the second embodiment of the present invention. The fluid delivery device 2' is an aerosol delivery device for delivering an aerosol as the fluid into the human or animal body. Specifically, the fluid delivery device 2 is a vibrating membrane aerosol delivery device comprising a vibrating membrane aerosol generator 24' for generating an aerosol (see Figure 2(b)). The vibrating membrane aerosol generator 24' comprises a perforated vibratable membrane (not shown).

The fluid delivery device 2' comprises a liquid reservoir 26' for receiving a liquid drug or medicament. Further, the device 2' includes a cap 28', such as a screw cap, with which the liquid reservoir 26' can be closed after filling the liquid drug or medicament into the reservoir 26'. The liquid drug or medicament filled into the liquid reservoir 26' comes into contact with the perforated vibratable membrane of the aerosol generator 24'. The membrane is provided with a plurality of openings or holes with diameters in the micrometer range that fully penetrate the membrane. The perforated membrane can be vibrated or oscillated, for example, by means of a piezoelectric element (not shown), such that the direction of the vibrations is perpendicular to the plane of the membrane. The plane of the membrane is arranged horizontally in the arrangement shown in Figures 2(a) and (b).

By inducing vibrations in the perforated membrane of the aerosol generator 24', the liquid drug or medicament contained in the liquid reservoir 26' is passed through the openings or holes of the membrane and aerosolised into a fluid chamber 4' of the fluid delivery device 2'. The fluid chamber 4' is formed at the other side, opposite the liquid reservoir 26', of the perforated membrane. A detailed description of this concept is given, for example, in US 5 518 179.

The fluid delivery device 2' comprises an oscillator 6' for imparting oscillations to at least a portion of the fluid received in the fluid chamber 4'. The oscillator 6' comprises a vibrator 8' and a resonator 10' (see Figure 2(b)).

As has been indicated above, in the fluid delivery device 2' of the second embodiment, the oscillator 6' is integrally formed with a remainder of the device 2'. Specifically, as is shown in Figure 2(b), the fluid chamber 4' forms a main body 12' of the resonator 10', defining an interior volume 14'. The resonator 10' has the main body 12', defining the interior volume 14', and a neck 16' which defines an inner space 18'. The neck 16' is connected to the main body 12' and in fluid communication with the interior volume 14'. As is schematically shown in Figure 2(b), both the interior volume 14' and the inner space 18' have an irregular shape.

The vibrator 8' is configured to impart vibrations to the main body 12' of the resonator 10'. The vibrator 8' of the device 2' of the second embodiment has substantially the same configuration as the vibrator 8 of the device 2 of the first embodiment. Hence, a detailed description thereof has been omitted.

The fluid delivery device 2' further comprises a mouthpiece or nosepiece 22' for adaptation to the respiratory system of the human or animal body. The aerosol as the fluid received in the fluid chamber 4' is transported outside the device 2' through the mouthpiece or nosepiece 22'.

A fluid flow of at least a portion of the fluid received in the fluid chamber 4' is induced by the respiration, i.e., the inhalation, of the patient or by one or more inlet valves (not shown) provided in the fluid delivery device 2'. The one or more inlet valves are configured to exploit the oscillations of a fluid, e.g., a gas, such as air, received in the interior volume 14' so as to generate a fluid flow for transporting aerosol as the fluid outside the device 2'. Alternatively, the fluid delivery device 2' may be provided with a different fluid conveying element, such as a pump or the like.

The oscillator 6' is configured to impart oscillations to the fluid flow.

Further, the fluid delivery device 2' comprises a control (not shown) for controlling the oscillator 6'. The control is configured to control operation of the vibrator 8' so as to impart vibrations to the main body 12' of the resonator 10' at a resonance frequency of the resonator 10' or at a frequency which is within ± 20% of a resonance frequency of the resonator 10'.

The oscillator 6' and the control together form an embodiment of the oscillator system of the present invention.

The configuration and the operation of the control of the device 2' of the second embodiment are substantially the same as the configuration and the operation, respectively, of the device 2 of the first embodiment. Hence, a detailed description thereof has been omitted.

The resonator 10', having the main body 12' and the neck 16', is a Helmholtz resonator. The resonator 10' may have a single resonance frequency or a plurality of resonance frequencies. The control may be configured to control operation of the vibrator 8' so as to impart vibrations to the main body 12' at one or more of the resonance frequencies of the resonator 10' or at one or more frequencies which are within ± 20% of a resonance frequency of the resonator 10'. In particular, the control may be configured to scan a frequency band comprising one or more of these resonance frequencies or one or more frequencies which are within ± 20% of a resonance frequency of the resonator 10'.

In operation of the fluid delivery device 2', a liquid drug or medicament is filled into the liquid reservoir 26' and the liquid reservoir 26' is closed with the cap 28'. Subsequently, the aerosol generator 24' is actuated, i.e., the perforated vibrating membrane thereof is vibrated, so as to aerosolise the liquid drug or medicament received in the liquid reservoir 26' into the fluid chamber 4'. A fluid flow is induced in at least a portion of the aerosol received in the fluid chamber 4', e.g., by the inhalation of the patient through the mouthpiece or nosepiece 22', by one or more inlet valves provided in the device 2' or by a different fluid conveying element, such as a pump or the like, and oscillations are imparted to the fluid flow by the oscillator 6'. For imparting these oscillations, operation of the vibrator 8' is controlled by the control so as to vibrate the main body 12', i.e., a fluid received in the interior volume 14' thereof, at a resonance frequency of the resonator 10' or at a frequency which is within ± 20% of a resonance frequency of the resonator 10'.

In this way, the aerosol can be efficiently delivered to regions of the human or animal body which otherwise are difficult to reach, in substantially the same manner as for the fluid delivery device 2 of the first embodiment.

The fluid delivery device 2' of the second embodiment is a hand-held and portable device enabling a particularly convenient and efficient aerosol delivery.

Figure 3 is a diagram showing measurement results of the pressure amplitude of the vibrations amplified by the resonator 10 of the fluid delivery device 2 of the first embodiment as a function of the frequency of the vibrations imparted to the main body 12 by the vibrator 8. As is indicated in Figure 3, the pressure amplitude is given in arbitrary units and the frequency is given in Hz.

The resonance frequency of the resonator 10, i.e., the frequency at which maximum amplification of the vibrations occurs, is approximately 45 Hz in this example (see Figure 3). As is further evident from Figure 3, the pressure amplitude can be significantly increased by suitably choosing the frequency of the vibrator 8.

Figure 4 is a diagram showing measurement results for the pressure amplitude of the vibrations amplified by the resonator 10 of the fluid delivery device 2 of the first embodiment as a function of the electric power applied to the vibrator 8. The electric power is given in W. The pressure amplitude has been normalised by dividing the measured values by respective pressure amplitudes measured for a conventional pulsating aerosol nebuliser. The pressure amplitude is thus given in Figure 4 in percent of the pressure amplitude of the conventional pulsating aerosol nebuliser. For the measurement shown in Figure 4, the vibrator 8 was operated to impart vibrations to the main body 12 at a constant frequency of 50 Hz.

As is shown in Figure 4, the pressure amplitude increases significantly with increasing electric power. Already at an electric power of approximately 1.5 W, pressure amplitudes of the order of those achievable with the conventional pulsating aerosol nebuliser are obtained (see the vertical dashed line in Figure 4). If the electric power is further increased to approximately 4.5 W, about 150% of the pressure amplitude of the conventional pulsating aerosol nebuliser are achieved.

Due to the low power consumption of the fluid delivery device 2, the oscillator 6 can be continuously operated for approximately 2 to 3 hours at a pressure amplitude of about 100% of the pressure amplitude of the conventional pulsating aerosol nebuliser using a AA type battery as the power source.

The fluid delivery device of the present invention thus allows for a fluid to be delivered into a human or animal body in an efficient manner, while providing a compact device configuration, reduced power consumption, a decreased noise level and greater flexibility.

## Claims

1. A fluid delivery device (2) for delivering a fluid into a human or animal body, wherein the fluid delivery device (2) comprises
a fluid chamber (4) for receiving a fluid;
an oscillator (6) for imparting oscillations to at least a portion of the fluid; and
a control for controlling the oscillator (6); wherein
the oscillator (6) comprises a vibrator (8) and a resonator (10),
the resonator (10) has a main body (12), defining an interior volume (14), and a neck (16),
the neck (16) is connected to the main body (12) and in fluid communication with the interior volume (14),
the vibrator (8) is configured to impart vibrations to the resonator (10), and
the control is configured to control operation of the vibrator (8) so as to impart vibrations to the resonator (10) at a resonance frequency of the resonator (10) or at a frequency which is within ± 20% of a resonance frequency of the resonator (10).

2. A fluid delivery device (2') for delivering a fluid into a human or animal body, wherein the fluid delivery device (2') comprises
a fluid chamber (4') for receiving a fluid;
an oscillator (6') for imparting oscillations to at least a portion of the fluid; and
a control for controlling the oscillator (6'); wherein
the oscillator (6') comprises a vibrator (8') and a resonator (10'),
the fluid chamber (4') forms a main body (12') of the resonator (10'), defining an interior volume (14'),
the resonator (10') has the main body (12') and a neck (16'),
the neck (16') is connected to the main body (12') and in fluid communication with the interior volume (14'),
the vibrator (8') is configured to impart vibrations to the resonator (10'), and
the control is configured to control operation of the vibrator (8') so as to impart vibrations to the resonator (10') at a resonance frequency of the resonator (10') or at a frequency which is within ± 20% of a resonance frequency of the resonator (10').

3. The fluid delivery device (2, 2') according to claim 1 or 2, further comprising a fluid conveying element for inducing a fluid flow of at least a portion of the fluid received in the fluid chamber (4, 4'), wherein the oscillator (6, 6') is configured to impart oscillations to the fluid flow.

4. The fluid delivery device (2, 2') according to any one of the preceding claims, wherein the fluid delivery device (2, 2') is an aerosol delivery device and the fluid is or contains an aerosol.

5. The fluid delivery device (2, 2') according to claim 4, further comprising an aerosol generator (24') for generating an aerosol.

6. The fluid delivery device (2, 2') according to any one of the preceding claims, wherein the vibrator (8, 8') comprises a vibratable membrane.

7. The fluid delivery device (2, 2') according to any one of the preceding claims, wherein the main body (12, 12') of the resonator (10, 10') comprises a vibratable membrane.

8. The fluid delivery device (2, 2') according to any one of the preceding claims, wherein the control is configured to control operation of the vibrator (8, 8') so as to impart vibrations to the resonator (10, 10') at a constant frequency, the constant frequency being a resonance frequency of the resonator (10, 10') or a frequency which is within ± 20% of a resonance frequency of the resonator (10, 10').

9. The fluid delivery device (2, 2') according to any one of claims 1 to 7, wherein the control is configured to control operation of the vibrator (8, 8') so as to impart vibrations to the resonator (10, 10') at a plurality of different frequencies, and at least one of the plurality of frequencies is a resonance frequency of the resonator (10, 10') or a frequency which is within ± 20% of a resonance frequency of the resonator (10, 10').

10. The fluid delivery device (2, 2') according to any one of the preceding claims, wherein the resonator (10, 10') has a single resonance frequency or a plurality of resonance frequencies.

11. The fluid delivery device (2, 2') according to any one of the preceding claims, wherein an inner cross-section of the neck (16, 16'), perpendicular to a fluid path direction (F) in the neck (16, 16'), is substantially constant along the fluid path direction (F) in the neck (16, 16').

12. The fluid delivery device (2) according to any one of the preceding claims, wherein the interior volume (14) of the main body (12) has a cylindrical shape or a spherical shape.

13. The fluid delivery device (2, 2') according to any one of the preceding claims, wherein a length of the neck (16, 16') in a fluid path direction (F) in the neck (16, 16') is in the range of 15 mm to 150 mm, preferably 25 mm to 75 mm.

14. The fluid delivery device (2, 2') according to any one of the preceding claims, wherein an inner diameter of the neck (16, 16'), perpendicular to a fluid path direction (F) in the neck (16, 16'), is in the range of 1.0 mm to 30.0 mm, preferably 5.0 mm to 20.0 mm.

15. The fluid delivery device (2, 2') according to any one of the preceding claims, wherein the size of the interior volume (14, 14') of the main body (12, 12') is in the range of 10 ml to 800 ml, preferably 50 ml to 400 ml.

16. The fluid delivery device (2, 2') according to any one of the preceding claims, wherein the resonator (10, 10') has at least one resonance frequency in the range of 10 Hz to 200 Hz, preferably in the range of 20 Hz to 100 Hz.

17. The fluid delivery device (2, 2') according to any one of the preceding claims, further comprising an adaptation element (22, 22') for adaptation to the respiratory system of a human or animal body.

18. The fluid delivery device (2, 2') according to any one of the preceding claims, wherein the oscillator (6) is removably attached to a remainder of the fluid delivery device (2), or the oscillator (6') is integrally formed with a remainder of the fluid delivery device (2').

19. The fluid delivery device (2, 2') according to any one of the preceding claims, wherein the vibrator (8) is removably attached to a remainder of the fluid delivery device (2), or the vibrator (8') is integrally formed with a remainder of the fluid delivery device (2').

20. A method of operating the fluid delivery device (2, 2') according to any one of the preceding claims, the method comprising
controlling operation of the vibrator (8, 8') so as to impart vibrations to the resonator (10, 10') at a resonance frequency of the resonator (10, 10') or at a frequency which is within ± 20% of a resonance frequency of the resonator (10, 10').

21. An oscillator system for the fluid delivery device (2, 2') according to any one of claims 1 to 19, wherein the oscillator system comprises
an oscillator (6, 6') for imparting oscillations to at least a portion of the fluid; and
a control for controlling the oscillator (6, 6'); wherein
the oscillator (6, 6') comprises a vibrator (8, 8') and a resonator (10, 10'),
the resonator (10, 10') has a main body (12, 12'), defining an interior volume (14, 14'), and a neck (16, 16'),
the neck (16, 16') is connected to the main body (12, 12') and in fluid communication with the interior volume (14, 14'),
the vibrator (8, 8') is configured to impart vibrations to the resonator (10, 10'), and
the control is configured to control operation of the vibrator (8, 8') so as to impart vibrations to the resonator (10, 10') at a resonance frequency of the resonator (10, 10') or at a frequency which is within ± 20% of a resonance frequency of the resonator (10, 10').
